(19) Europäisches Patentamt
European Patent Office
Office européen des brevets

(11) **EP 4 748 295 A1**

(12) **EUROPEAN PATENT APPLICATION**

(43) Date of publication:
**27.05.2026 Bulletin 2026/22**

(21) Application number: **24215102.5**

(22) Date of filing: **25.11.2024**

(51) International Patent Classification (IPC):
**A61B 3/10** (2006.01)  **A61B 3/12** (2006.01)

(52) Cooperative Patent Classification (CPC):
**A61B 3/12; A61B 3/1025**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Optos plc**
**Dunfermline, Scotland KY11 8GR (GB)**

(72) Inventor: **MUYO, Gonzalo**
**Dunfermline, Scotland, KY11 8GR (GB)**

(74) Representative: **Hoffmann Eitle**
**Patent- und Rechtsanwälte PartmbB**
**Arabellastraße 30**
**81925 München (DE)**

(54) **ABERRATION CORRECTION IN AN OPHTHALMIC IMAGING INSTRUMENT**

(57) An ophthalmic imaging instrument comprising a light source emitting a beam of light; a polygon scanning mirror comprising a plurality of reflecting facets; a driver arranged to rotate during operation said polygon scanning mirror, such that each facet reflects the beam of light at a varying angle; an optical system arranged to guide the beam reflected from the polygon scanning mirror towards a fundus of an eye of a subject through a cornea of the eye and to guide returning light back towards the polygon scanning mirror; an aberration correction element being located between the polygon scanning mirror and the optical system, the aberration correction element comprising an elongate portion of varying thickness supported by a carrier; and a mask arranged on the carrier and to block light in a periphery of the elongate portion of the aberration correction element.

Fig. 2

## Description

Technical field

The present invention relates to correcting aberration in an ophthalmic imaging instrument. More particularly but not exclusively, the present invention relates to an aberration correction element that guides a beam of light reflected from and to a polygon scanning mirror and to reducing the pick-up of undesired beam components by a light detector of the ophthalmic imaging instrument.

Background

Ophthalmic imaging instruments are widely used to image a patient's eye in order to assess the health of the eye. Such instruments comprise a light source and a controller that is arranged to control the light source to generate a beam of light of a target optical power. The beam of light is deflected by one or more scan relay elements so as to cover an imaging area by means of a typically parallel arrangement of a plurality of scanning lines. The scan relay element(s) usually comprise a polygon scanning mirror and one or more scanning galvos. The former polygon scanning mirror comprises a plurality of reflecting facets that are arranged along an outer circumference of a rotating body. A driver is operable to rotate the body of the polygon scanning mirror, such that each facet successively reflects an incident beam of light at a varying angle. Thus, during the passing of one individual facet, the beam of light is deflected through the varying angle. After completion of such a deflection cycle, the next facet substantially repeats this process and so do all other facets.

This repeated deflection by the polygon scanning mirror forms the basis for at least one scanning direction by means of providing a plurality of parallel scanning lines. A further scanning element, such as an already mentioned galvo, can then further deflect the beam in another direction so as to displace the scanning lines from each other on the target and to ultimately cover a two-dimensional scanning area. At least in some imaging modalities, the light from the scanning beam is scattered back from the target tissue which includes in the case of ophthalmic imaging primarily parts of the human eye such as, for example, the ocular fundus or retina. In general, the light thus travels back along the incident light path so as to be ultimately detected by a detector in the form of a light sensor, converted into an intensity signal, and processed to form the image. Especially the latter signal processing is implemented by means of computing resources that compile individual images from corresponding sets of line scans.

Where the target tissue is the fundus of the eye, the light from the scanning beam passes through the cornea of the eye and reaches the retina as an imaging target. Light is reflected by the retina and is led back toward the detector by help of the optical elements within the instrument. However, the light wich is reflected by the retina first emerges from the eye via the patient's pupil which, in a sense, froms a larger exit aperture as compared to the input beam which is typically a laser beam with a relatively small spot size. In addition to this, the cornea (e.g. the outer surface of the cornea) may specularly reflect some of the scanning beam back towards the detector, thereby contributing to artifacts in the image. Other image artefacts may be similarly formed from some of the scanning beam specularly reflecting back from optical elements within the instrument. In all, this results in that the cross-section of the incoming beam (to the detector) is usually larger than the cross-section of the outgoing (from the light source) scanning beam. Amongst others, the incoming beam may comprise undesired components as a result of reflections at interfaces other than the main imaging target. This may have a particular impact when optical elements are involved which have dimensions that are similar to the cross-section(s) of the beam(s) of light they guide.

Therefore, there is a need for an improved structure and configuration of an ophthalmic imaging instrument that takes into account the effect of dimensions of beam cross sections relative to the characteristics of the optical elements involved. With this, there is a particular need to suppress or eliminate any image artifacts related to the mentioned back-reflections of the scanning beam, whilst keeping system complexity at an acceptable level.

Summary

Problems are solved and objects are met by the subject matter of the independent claim. Further preferred embodiments are defined in the dependent claims.

According to one embodiment of the present invention, there is provided an ophthalmic imaging instrument comprising a light source emitting a beam of light, a polygon scanning mirror comprising a plurality of reflecting facets, a driver arranged to rotate during operation said polygon scanning mirror, such that each facet reflects the beam of light at a varying angle, an optical system arranged to guide the beam reflected from the polygon scanning mirror towards a fundus of an eye of a subject through a cornea of the eye and to guide returning light back towards the polygon scanning mirror, an aberration correction element being located between the polygon scanning mirror and the optical system, the aberration correction element comprising an elongate portion of varying thickness supported by a carrier; and a mask arranged on the carrier and to block light in a periphery of the elongate portion of the aberration correction element.

Brief Description of the Drawings

Embodiments of the present invention, which are presented for better understanding of the inventive concepts, but which are not to be seen as limiting the invention, will now be described with reference to the figures in which:

| | |
|---|---|
| Figure 1 | shows a schematic view of an ophthalmic imaging instrument according to a general device embodiment of the present invention; |
| Figure 2 | shows a schematic view of an ophthalmic imaging instrument according to a device embodiment of the present invention; |
| Figures 3A and 3B | show a schematic front and side view of an aberration correction element comprising an elongate portion of varying thickness as part of an ophthalmic imaging instrument of an embodiment of the present invention; |
| Figures 4A and 4B | show schematic views of an aberration correction element in conjunction with a carrier as part of an ophthalmic imaging instrument of an embodiment of the present invention; |
| Figure 5 | shows a schematic view of an aberration correction element in conjunction with a mask as part of an ophthalmic imaging instrument of an embodiment of the present invention; and |
| Figure 6 | shows a schematic view of a placement and location of an aberration correction element as part of an ophthalmic imaging instrument of an embodiment of the present invention. |

It should be understood that some of the drawings may not necessarily be shown to scale, unless otherwise indicated. In certain instances, details that are not necessary for an understanding of the disclosure or that render other details difficult to perceive may have been omitted. It should be understood, of course, that the disclosure is not necessarily limited to the particular examples or embodiments illustrated or depicted herein.

Detailed Description

The present inventors have recognised that due to an enlarged cross-section of the beam of light that is reflected back from the imaging target, some beam components may be incident to parts of optical elements that in turn produce further undesired stray light effects. For example, an optical element concerned may be an aberration correction element having an elongate portion of varying thickness. In this way, such a portion may protrude from or indent into a carrier and thus forming side walls. Parts of a larger beam cross-section may be thus incident to such sidewalls which can then act as a further surface undesirably reflecting any beam components incident thereto. Accordingly, the inventors have devised a mask which blocks such portions of the beam from eventually reaching a detector of the ophthalmic imaging instrument. The mask may thereby at least partially suppress image artefacts in images of the fundus which would otherwise result from the detection of these back-reflections. The quality of images generated by the ophthalmic imaging instrument may thus be greatly improved.

Example embodiments herein will now be described with reference to the accompanying drawings.

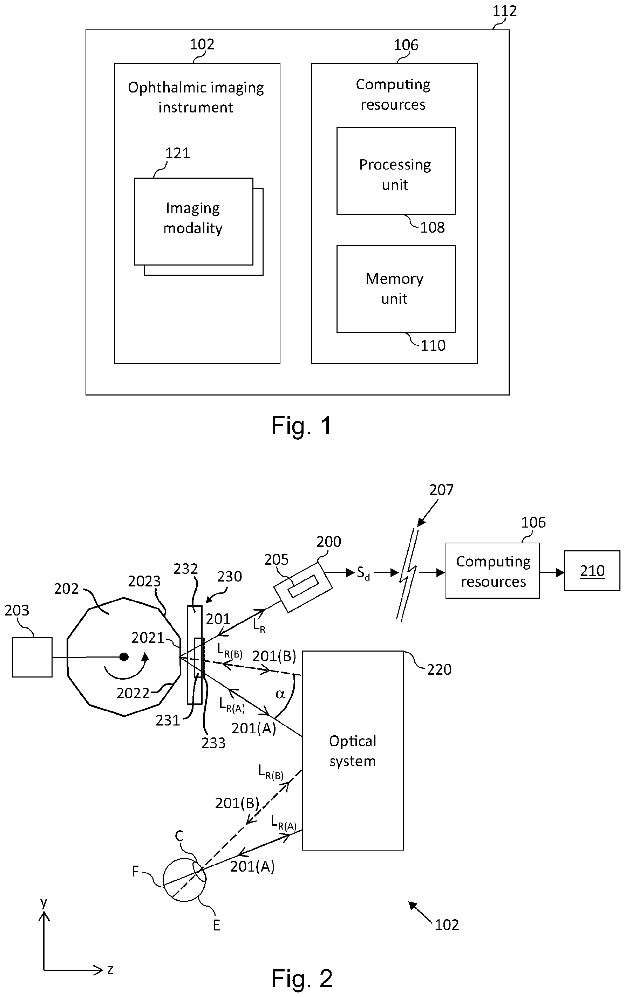

Figure 1 shows a schematic view of an ophthalmic imaging instrument 102 according to a general device embodiment of the present invention. Specifically, there is shown an ophthalmic imaging system 112 which includes the ophthalmic imaging instrument 102 providing at least one imaging modality 121. For example, the ophthalmic imaging instrument 102 can assume the form of a scanning laser ophthalmoscope (SLO) or an optical coherence tomography (OCT) imaging instrument. The imaging modality 121 can then be understood as an operation mode in which the respective instrument is operated. Generally, the ophthalmic imaging instrument 102 can be operated in a plurality of modes, providing a plurality of corresponding imaging modalities. The ophthalmic imaging system 112 may comprise or have access to (e.g. via a wired or wireless connection) computing resources 106 which, in turn, comprise a processing unit 108 and a memory unit 110. The components of the ophthalmic imaging system 112 including the ophthalmic imaging instrument 102 and the computing resources 106 may be housed inside a common housing so that the system 112 thereby forms the ophthalmic imaging instrument 102, such as an ophthalmoscope. In this way, the ophthalmic imaging instrument 102 has access to, or itself comprises, the computing resources 106. In some embodiments, the computing resources 106 may be located external to the instrument 102 within respective different housings. In some embodiments, any of the components may be located in a different housing from the rest of the components.

The computing resources 106 control the ophthalmic imaging instrument 102 to operate in the selected imaging modality 121 and the computing resources have at least one processing unit 108 such as a central processing unit (CPU) and/or graphics processing unit (GPU), and a memory unit 110 that stores instructions that, when executed by the at least one processing unit 108, causes the processing unit 108 to perform one or more methods and functions described herein. In embodiments of local computing resources or local physical components of a computing device, the resources may include a processor, such as CPU and/or GPU, a system memory, and a system bus that couples the system memory to the CPU/GPU. The system memory may include a random access memory ("RAM") and a read-only memory ("ROM").

A basic input/output ("I/O") system containing the basic routines that help to transfer information between elements within the computing device, such as during startup, is stored in the ROM. The computing resources may further include a mass storage device, which is able to store software instructions and data. The mass storage device may be connected to the CPU/GPU through a mass storage controller connected to the system bus. The mass storage device and its associated computer-readable data storage media may provide non-volatile, non-transitory storage for the computing resources 106. Although the description of computer-readable data storage media contained herein refers to a mass storage device, such as a hard disk or CD-ROM drive, it should be appreciated by those skilled in the art that computer-readable data storage media can be any available non-transitory, physical device or article of manufacture from which the device can read data and/or instructions. The mass storage device is an example of a computer-readable storage device.

Computer-readable data storage media include volatile and non-volatile, removable and non-removable media implemented in any method or technology for storage of information such as computer-readable software instructions, data structures, program modules or other data. Example types of computer-readable data storage media include, but are not limited to, RAM, ROM, EPROM, EEPROM, flash memory or other solid-state memory technology, CD-ROMs, digital versatile discs ("DVDs"), other optical storage media, magnetic cassettes, magnetic tape, magnetic disk storage or other magnetic storage devices, or any other medium which can be used to store the desired information and which can be accessed by the computing device.

The computing resources 106 may operate in a networked environment using logical connections to remote network devices through a network, such as a local network, the Internet, or another type of network. The computing resources 106 may connect to the network through a network interface unit connected to the system bus. The network interface unit may also connect to other types of networks and remote computing systems. The computing resources 106 may include an input/output controller for receiving and processing input from a number of other devices, including a touch user interface display screen, or another type of input device. Similarly, the input/output controller may provide output to a touch user interface display screen, a printer, or other type of output device. As mentioned above, the mass storage device and the RAM can store software instructions and data. The software instructions may include an operating system suitable for controlling the operation of the computing resources 106. The mass storage device and/or the RAM may also store software instructions, that when executed by the CPU/GPU, cause the computing resources 106 to provide the functionality discussed in this document, including the methods described herein and shown in the figures.

In some embodiments, the imaging modality 121 may be provided as an operation mode of the already mentioned SLO, which may use confocal laser scanning microscopy for diagnostic imaging of the retina of the eye. The imaging may be two-dimensional (2D) imaging and may use a laser light beam to scan across the retina in a raster pattern to illuminate successive elements of the retina, point-by-point. The light reflected from each retinal point may be captured by a photomultiplier. The output of the photomultiplier may be recorded and displayed in a digital format. In this manner, the imaging modality in the SLO may be able to produce high-contrast, detailed images of the retina. In some embodiments, images are captured sequentially by one imaging modality and by at least a further imaging modality.

Figure 2 shows a schematic view of the ophthalmic imaging instrument 102 according to a device embodiment of the present invention. As shown, the ophthalmic imaging instrument 102 comprises a light source 200 arranged to emit a beam of light 201 as a scanning beam. A polygon scanning mirror 202 acts a first scanning element (or, in other terms, a first scan relay) which comprises a plurality of reflecting facets 2021, 2022, .... In some embodiments, the beam 201 may have a cross-sectional area which is fraction of the area of the facets (e.g. less than 0.1% or 1% of the area of each of the facets.

The polygon scanning mirror 202 is arranged to reflect the beam 201 towards an optical system 220 and to reflect light $L_R$ returning from the eye E towards the detector 205, as later described. As shown, the beam 201 may, upon incidence with the polygon scanning mirror 202, be travelling with a component in the direction opposite to the positive y-direction. In the present embodiment, the polygon scanning mirror is a regular convex polygon with 12 rectangular facets, each having a length defined by the lengths of two edges parallel to the plane of rotation of the polygon scanning mirror 202 and a width (i.e. in the x-direction) defined by the lengths of two edges orthogonal to this plane. However, other numbers of facets (e.g. 16 facets or at least four or five facets), shapes of facets or geometries of polygon may instead be used.

A driver 203 is arranged to rotate during operation said polygon scanning mirror 202 (in the plane of rotation of the polygon scanning mirror 202, i.e. the y-z plane) such that each facet 2021, 2022, ... reflects the beam of light 201 at a varying angle α (when the facet is rotated over a predetermined range of angular positions). The driver 203 may, for example, be an electric motor connected to the polygon scanning mirror 202 and controlled by a controller of the ophthalmic imaging instrument 102 (which may be the computing resources 106). As illustrated, the path of the scanning beam 201 is shown in a one-dimensional (1D) scan produced during example anti-clockwise rotation (shown by the curved arrow) of the polygon scanning mirror 202. Path "A" is an example of the scanning beam 201 reflected from a respective facet 2021 of the polygon scanning mirror 202 at a starting point during rotation, i.e. when the facet 2021 is moved into the path of the beam 201 from the light source 200. Path "B" is an example of the scanning beam 201 reflected from the respective facet 2021 of the polygon scanning mirror 202 at a later point in time further down the rotation when the facet 2021 is moved out of the path of the beam 201 from the light source 200.

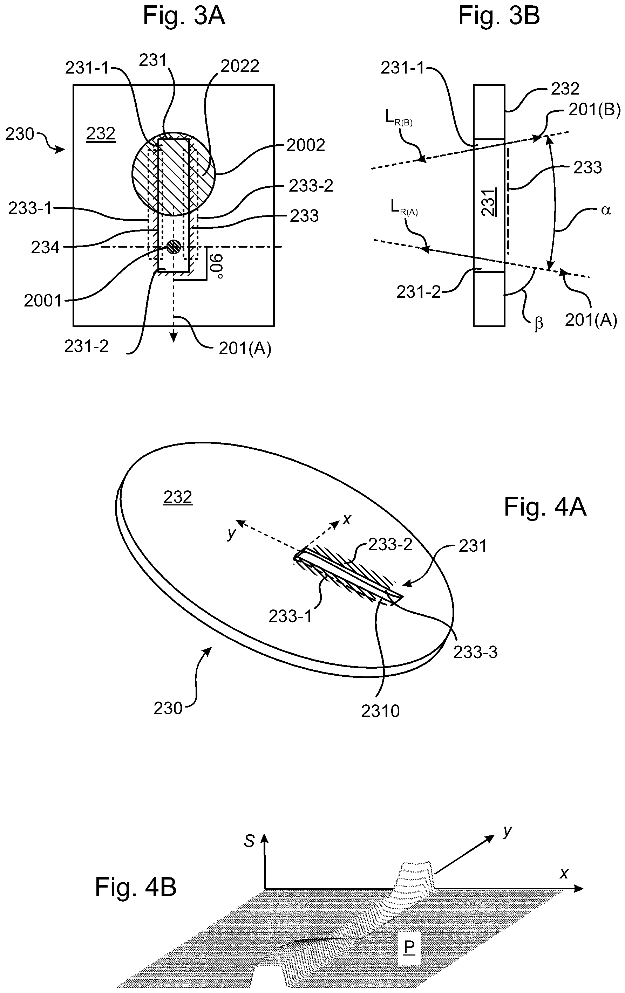

In this way, the polygon scanning mirror 202 reflects the beam 201 at (or through) a varying angle $\alpha$ from the active facet (i.e. the facet presently reflecting the beam 201) as the beam 201 moves from one edge of the facet defining the facets length to the other. As the facet 2021 moves out of reach of the beam 201, the adjacent facet 2022 (i.e. the neighbouring/bordering facet to the facet 2021 in the opposite rotational direction to that of the polygon scanning mirror 202) repeats substantially the described reflection process such that each facet successively reflects the beam 201 at the varying angle $\alpha$ in turn. This varying angle $\alpha$ varies as governed by the length and rotation of the respective facet as its respective orientation relative to the incident beam 201 determines the reflection angle. The ophthalmic imaging instrument 102 further comprises the optical system 220 arranged to guide the beam of light 201 that is reflected from the polygon scanning mirror 202 (i.e. the beam 201 reflected at the varying angle $\alpha$) towards a fundus F of an eye E of a subject through a cornea C of the eye E. Examples of components and optical elements within the optical system 220 are described later with reference to Figure 4.

The optical system 220 is further arranged to guide returning light $L_R$ back from the fundus F towards the polygon scanning mirror 202. The returning light $L_R$ may be light scattered from the eye E in, for example, the colour red-green or the colour red-green-blue imaging modality or may be, or comprise, light emitted from the eye E in, for example, the AF imaging modality. This return light $L_R$ may follow the same optical path as the beam 201 and so may be guided by the optical system 220 towards the polygon scanning mirror 202 along optical paths corresponding to the reflection of the beam 201 at the varying angle $\alpha$, as later described. The returning light $L_R$ is thus guided by the optical system 220 to the active facet and this active facet reflects the returning light $L_R$ towards a detector 205 along the optical path of the beam 201 so as to form an image 210 of the eye E. As already mentioned, the returning light beam $L_R$ usually has a larger cross-section as compared to the (incident) beam of light 201, mainly as a result of the returning light emerging through a comparatively larger exit aperture (pupil) after being reflected from the imaging target (e.g. retina).

As the current active facet moves out of reach of the beam 201, the adjacent facet becomes the active facet and repeats substantially this described reflection process of the returning light $L_R$ such that each facet reflects the returning light $L_R$ towards the detector 205 along the optical path of the beam 201, in turn. Thus, successive scan lines in the image 210 may be formed by using, for example, an orthogonal scanning element, as later described. In some embodiments, the returning light $L_R$ from the eye E may be a light beam of a diameter which is greater than that of the beam 201 and which is greater than the larger of the width and length of each of the facets, although it would be appreciated by those skilled in the art that this depends on the components and optical elements in the optical system 220, the characteristics of the beam 201 and the imaging modality 121, for example.

The ophthalmic imaging instrument 102 further comprises an aberration correction element 230 which is located between the polygon scanning mirror 202 and the optical system 220. This aberration correction element 230 is generally provided for - at least in part - compensating the aberration that originates from the light passing through a patient's pupil. For example, the aberration correction element 230 can be in the form of a phase mask which is approximately conjugate with the patient's pupil. As the cross section (spot size) of the return beam on the aberration correction element 230 is usually larger than that of the incident scanning beam, a differentiation between compensating only the incident beam and compensating both the incident and returning beam can be made: Assuming that the cross section of the returning beam is roughly the size of the patient's pupil, a phase mask with an elongate portion of altering aberration can substantially only correct the incident beam, as practically all of the incident beam with its relatively small cross-section passes through the relatively narrow slot of such an elongate portion, while the returning light with its relatively large cross section passes through the phase mask for most of its part in sections of the phase mask outside the active elongate portion (see also exemplary cross-sections 2001 & 2002 as shown and described in conjunction with Fig. 3A).

It is noted that at this stage in the optical path, the beam of light varies its direction over time, as the polygon scanning mirror imposes a periodic scanning onto the outgoing beam of light originating from the light source(s). As also the incoming beam of light travels substantially along the same path, this beam varies direction as well, which is only compensated for by the polygon scanning mirror again which directs the incoming beam at a substantially constant direction toward the detector(s). As a result, the beams of light may be incident at varying positions and/or with varying angles at optical elements of the optical system, which, in turn, may result in the beam of light being subject to varying aberration with the varying direction. The aberration correction element 230 thus comprises an elongate portion 231 as already mentioned ans which covers a path of an intersection point of the beam of light as it varies direction and/or angle. Such an elongate portion 231 may comprise a varying thickness for imposing a varying optical depth and, with this, a varying phase shift for compensating aberration. A more detailed embodiment of such an aberration correction element is disclosed in conjunction with Figures 4A & 4B.

The ophthalmic imaging instrument further comprises a mask 233 arranged on a carrier 232 and to block light in a periphery of the elongate portion 231 of the aberration correction element 230. It is noted that in the side view of Figure 2, the mask 233 is depicted as a dashed line as it certainly does not block light which is supposed to be guided towards and back from the imaging target (eye). The effectively larger beam cross-section of the return light may result in light being incident into parts of the aberration correction element 230 which may cause further reflections and deviations from a target light path within the system. As such stray light may eventually reach the detector 205 so as to form image artifacts in the image 210, the ophthalmic imaging instrument comprises a mask (or light blocker) arranged to block any portion of the beam of light that is disadvantageously affected by a periphery of the elongate portion, or parts thereof, of the aberration correction element.

Figures 3A and 3B show a schematic front and side view of an aberration correction element comprising an elongate portion of varying thickness as part of an ophthalmic imaging instrument of an embodiment of the present invention. Specifically, an aberration correction element 230 is shown in a front view and resting in a plane that may form a substantially right angle with a beam of light at least in one dimension. That is, the beam of light 201(A) may form a substantially right angle of 90° with the plane of the element 230 as shown in Figure 3A, while following a varying angle $\beta$ relative to that plane due to the scanning motion with the varying angle $\alpha$ as shown in Figure 3B. The varying angle $\alpha$ is provided by the rotation of the polygon scanning mirror, whose facets each reflect the beam of light incident from the light source and incident from the imaging target at a varying angle.

The aberration correction element 230 comprises an elongate portion 231 in a carrier 232 and that is to cover the likewise elongate area that the beam of light covers when crossing the plane of the aberration correction element 230. That is, the elongate area 231 is related to the target path along which the point moves at which the beam of light crosses a plane of aberration correction element 230. For example, a first end 231-1 of the elongate portion 231 is located such that a beam of light at one extremum of the scanning motion can be still guided through the elongate portion 231. This may correspond to the beam of light 201(B) as described in conjunction with Figure 2. A second end 231-2 of the elongate portion 231 can then be located such that the beam of light at the other extremum of the scanning motion can be still guided through the elongate portion 231. This may, in turn, correspond to the beam of light 201(A) as described in conjunction with Figure 2.

As already explained, the beam of light 201 is first propagating toward the eye (E) of a subject as an outgoing beam 201(A), 201(B), reflected at parts of the eye (E) as an imaging target and then guided back as a reflected beam of light $L_{R(A)}$, $L_{R(B)}$. However, the respective cross-sections of the outgoing and incoming beam may be different. Usually, the outgoing beam of light 201(A/B) may have a relatively smaller first cross-section 2001 at a plane of the aberration correction element 230, while the incoming beam of light $L_{R(A/B)}$ may have a relatively greater second cross-section 2002 at the plane of the aberration correction element 230. While the first cross-section 2001 may be well accommodated within the elongate area 231, the second cross-section 2002 may comprise parts and beam components that overlap with elongate area 231. As a result, light may be incident to parts of the aberration correction element 230 that can in turn cause further undesired reflections and deviations from a target propagation part, as explained in greater detail in conjunction with Figures 4A & 4B. This, in turn, may lead to the pick-up of undesired beam components at the detector which may ultimately lead to image artifacts and distortions.

For avoiding such pick-up and to substantially suppress the generation of stray light related to the mentioned effects, a mask is provided on the carrier 232. The mask may be arranged in a periphery 233 of the elongate portion 231 of the aberration correction element 230. It is noted that in the shown schematic of Figure 3A, the reference numeral 233 may denote the periphery and the mask, as the latter is arranged to the extend of the former. The periphery 233 of the elongate portion 231 may comprise at least areas 233-1, 233-2 which are adjacent to the longitudinal sides of the elongate portion 231. These areas may overlap with the longitudinal sides or may extend only from the elongate portion away onto the carrier 232. In other words, at least the sides along the major axis (i.e. length or also y-direction) of the aberration correction element 230 may be considered by placing the mask (and blocking light) especially in such areas. However, the periphery may also comprise areas which cover side walls along the longitudinal sides of said elongate portion 231 preferably sidewalls of an elongate portion 231 that protrudes from and/or indents into the carrier 232. In other words, in case that the longitudinal sides of an elongate portion also include side walls as the elongate portion protrudes from and/or indents into a carrier, edge parts and/or sidewalls in relation to the elongate portion may be preferable regions and areas for which a mask is provided that blocks light to be reflected. The areas 233-1, 233-2 may be generally located in a central region in the longitudinal direction of the elongate portion, so as to accommodate any coating, blocking elements, or other forms of a mask. In this way, the significant central region is covered, while their central arrangement can facilitate forming, pacing and/or affixing the mask to the carrier.

This mask may comprise one or more blocking elements or a shadowing and/or opaque coating(s) on the relevant regions, parts and surfaces of the carrier 232. The latter coatings, e.g. in the form of an anti-reflective coating, may specifically apply to the mentioned edge parts and/or side surfaces in relation to portions that protrude from or indent into a carrier. In this way, light incident into the periphery of the elongate portion 231, such as parts of the larger light cross-section 2002, is effectively absorbed and thus prevented to generate any undesired reflections, and, with this, undesired stray light that may ultimately reach the detector(s) so as to produce imaging artifacts and distortions. However, the mask 233 does preferably only extend over the areas of the mentioned periphery of the elongate portion so as not to block the remainder of the return light with its relatively large cross section (i.e. light should be allowed to return through the area 2022).

Figures 4A and 4B show schematic views of an aberration correction element in conjunction with a carrier as part of an ophthalmic imaging instrument of an embodiment of the present invention. Specifically, the aberration correction element 230 is shown in the exemplary form of a static aberration correction element comprising a transmissive phase mask 2310 in a substantially rectangular shape so as to cover the corresponding elongate portion 231. This portion 231 is shown with a major axis along its length (y-direction) and a minor axis along its width (x-direction). The shape of the transmissive phase mask 231 follows generally a varying thickness and the phase mask is as such supported by or in the carrier 232. The thickness or depth 5 is spatially variant along the major axis and the minor axis of the mask 2310 and may be defined by at least one predetermined mathematical function, comprising:

$$S(x, y) = \sum_{i}^{N} a_i p_i(x, y),$$

where N is the number of polynomial coefficients in the series and $a_i$ is the coefficient ith on the polynomial term $p_i$. The polynomials are a power series in x and $y$. The first term is x, then y, then $x*x$, $x*y$, $y*y$, etc. In an example, N = 20 and the coefficients (divided by a normalization radius of R = 100 mm, so they are dimensionless) are: $a_1 = 0$, $a_2 = 1.515$, $a_3 = 9.981$, $a_4 = 0$, as = 15.486, $a_6 = 0$, $a_7 = -2342.830$, $a_8 = 0$, $a_9 = -635.766$, $a_{10} = 1026163.828$, $a_{11} = 0$, $a_{12} = -30279.492$, $a_{13} = 0$, $a_{14} = -5695.243$, $a_{15} = 0$, $a_{16} = 23929093.583$, $a_{17} = 0$, $a_{18} = -145044.106$, $a_{19} = 0$, $a_{20} = -14564.76025249$. This varying thickness is schematically shown in Figure 4B as a plot and refers to a varying thickness relative to some normal thickness appearing as the plane P. This plane P may not be equivalent to a surface of the carrier, as the elongate portion may be well etched into the carrier so that even the thickest parts remain within the carrier thickness and no part of the elongate portion may extend beyond a surface of the carrier 232.

In this embodiment, the depth (thickness) of the transmissive phase mask 2310 varies along the major axis, or length, of the mask with a maximum sag (peak-to-valley depth) of the mask being approximately 110 microns. The transmissive phase mask 2310 has a width of the approximately 1.5 mm and a length of approximately 12 mm. The transmissive phase mask 2310 may comprise optical glass with a spectral transmission extending from the visible portion to the near infrared portion of the electromagnetic spectrum. In general, the phase mask may be formed from a transparent material, preferably optical glass. In this way, the transmissive phase mask 2310 may be formed in the carrier 232 in the form of a glass substrate. The transmissive phase mask 2310 and generally the elongate portion of varying thickness may thus not only supported by the carrier 232 but also formed in that carrier 232 such as to form one piece. The latter configuration may substantially facilitate manufacturing of the aberration correction element 230 in an efficient and reproducible manner and the elongate portion of varying thickness can be etched into a substrate of the carrier 232.

The transmissive phase mask 2310 has a depth which varies along the major axis and the minor axis of the mask and therefore provides a spatially variant depth and corresponding spatially variant optical properties, including refraction, along the major and minor axes of the mask. As the incident light beam is scanned through the transmissive phase mask, the mask modifies the phase of the light beam by refraction. Phase characteristics of the mask which are consequential of the depth of the mask are imposed onto the phase of the light beam. As the depth of the mask varies along the major and minor axes of the mask, the phase modification of the incident light beam varies through two orthogonal axes of the light beam. This variation of the phase modification of the scan of the incident light beam may be advantageous for addressing at least some of the changing aberration which the scan of the light beam is subject to as it passes through the remainder of the optical system and the ophthalmic imaging instrument.

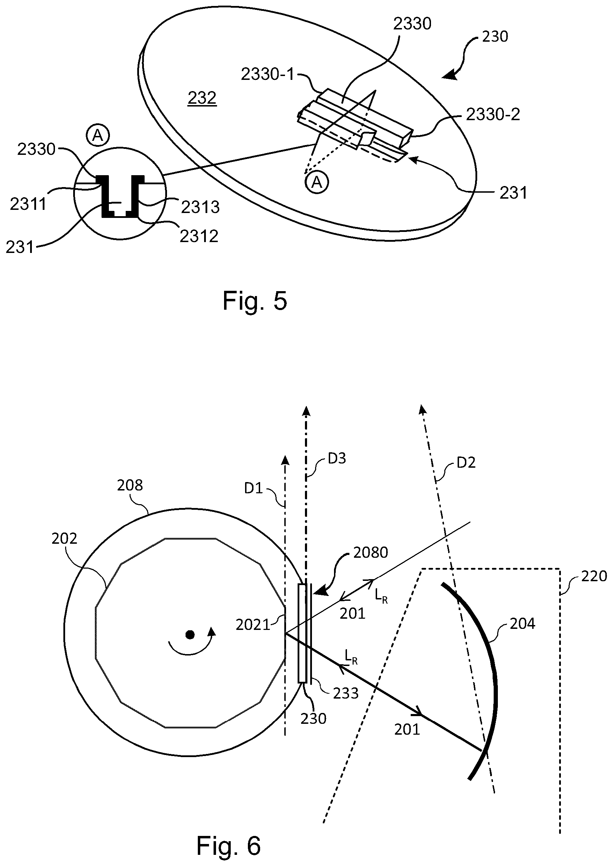

For example, scan compensation elements of the optical system, for example in the form of curved slit mirrors, may provide for a changing aberration along their respective major and minor axes. By defining a changing depth of the transmissive phase mask and locating the major axis of the mask substantially parallel to the major axis of such a scan compensation element, allows the mask to provide correction of the changing aberration as this is described in some greater detail in conjunction with Figure 5. The shape of the transmissive phase mask 2310 may thus modify the phase of the scan of the light beam such that after being propagated through the ophthalmic imaging instrument it comprises a substantially collimated and aberration-free beam of light at the apparent point source at the pupillary point of the eye which is transferred into the eye (E) and is focused by the eye onto a sharp spot (approximately 20 microns or less) for substantially every scan point of the field of view of the object and imaging target plane. Aberration which causes blurring and dimming can thus be reduced or avoided and this results in retaining the desired spatial information at substantially all portions of an image.

In the shown embodiment, the mask is provided in the form of a coating on relevant surfaces. Specifically, there may be provided a coating 233-1 and/or 233-2 arranged on the carrier 232 in the periphery of the elongate portion 231. These regions may refer to opposite faces of the carrier 232, so that a coating 233-1 may be provided for blocking and absorbing light that is incident from one side (facing top side in the Figure), while coating 233-2 may be provided for blocking and absorbing light that is incident from the other side (far side in the Figure). Further, coating 233-3 may be provided on any sidewalls in relation to the elongate portion 231 so as to likewise avoid or at least reduce reflections of light. Preferably, the mask, in the form of a coating and/or blocking element(s) - extends only along a longer direction of the elongate portion, i.e. the y-direction as shown in Figure 4A.

Figure 5 shows a schematic view of an aberration correction element in conjunction with a mask as part of an ophthalmic imaging instrument of an embodiment of the present invention. In the shown embodiment, an aberration correction element 230 is shown which is similar to or identical to the elements 230 described in conjunction with Figures 4A and 4B. However, the mask in this embodiment is provided by one or more blocking elements 2330 arranged in the periphery of the elongate portion and, more specifically, in the vicinity or adjacent to a side face of the elongate portion 231 (it is noted that one element 2330 is shown only in part so as to permit view to other relevant elements of the Figure). The blocking elements 2330 may be provided in the form of bars or slabs and are preferably made from a light absorbing material. However, the blocking elements 2330 may also comprise an opaque, antireflective and/or light absorbing coating. It is noted that the blocking elements 2330, as generally also the periphery of the elongate portion, do not need to necessarily extend along the entire long length of elongate portion as indicated by the ends 2330-1 and 2330-2 so that the mask extends only along a length that is shorter than the elongate portion in the longer direction. This may advantageously block any undesired light components but may still allow for a larger part of light returning from the eye E toward the detector through the carrier which may be transparent in all areas other the periphery that is covered by the mask or respective mask elements. In one example, the blocking elements 2330-1, 2330-2 may assume a form that covers the sidewalls 2313 of the elongate portion 231 and the corners/edge 2311 toward a main surface of the carrier 232 and the corners/edge 2312 toward a bottom surface of the elongate portion 231. The bottom surface may be formed by the elongate portion 231 indenting into the carrier 232 in the shape of a trough, as seen in the inset depicting the cross section of the elongate portion 231 and the blocking elements 2330-1, 2330-2 in a plane (A). In a way, the blocking elements 2330-1, 2330-2 may thus assume a form in which their respective cross-sections comprise two L-shape portions (such as to combine effectively to some sort of an S- or Z-like shape). This may allow for manufacturing and forming the blocking elements separately as a simple body via efficient techniques such as die-casting, extrusion, etc., which can then be affixed to the relevant parts of the phase mask and its elongate portion.

Figure 6 shows a schematic view of a placement and location of an aberration correction element as part of an ophthalmic imaging instrument of an embodiment of the present invention. In the embodiments, the aberration correction element 230 is located between the polygon scanning mirror and the optical system of the ophthalmic imaging instrument. Therefore, also in the present specific embodiment, the location of the aberration correction element 230, for example in the form of a transmissive phase mask, within the ophthalmic imaging instrument is between the polygon scanning mirror 202 and the optical system 220. As shown the polygon scanning mirror 202 is provided in a mirror housing 208 which may not only protect the mirror from dust, but also from other stray light that may cause any undesirable imaging artefacts when reaching the detector(s). The mirror housing 208 may provide for an opening 2080 on which the aberration correction element 230 is placed so that this may be the only optical access to the mirror facets 2021,.... Preferably, the mask 233 further limits this access to the elongate region 231 as the active optical element for correcting aberration.

The optical system 220 may comprise one or more scan compensation element(s) in the form of, for example, a curved slit mirror 204. Such scan compensation elements may provide for an extension so as to consider the varying directions of incident light beams. In the shown embodiment, the polygon scanning 202 deflects the beam of light 201 in a first plane indicated by line D1. The scan compensation element 204 is configured in a second plane indicated by line D2. Preferably, the lines D1 and D2 reside in the same plane. Preferably, also a major axis D3 of the aberration correction element 230 resides in that same plane. For example, the major axis D3 coincides with the major axis as described in conjunction with Figures 4A & 4B with the y-direction.

While this specification contains many specific embodiment details, these should not be construed as limitations on the scope of any inventions or of what may be claimed, but rather as descriptions of features specific to particular embodiments described herein. Certain features that are described in this specification in the context of separate embodiments can also be implemented in combination in a single embodiment. Conversely, various features that are described in the context of a single embodiment can also be implemented in multiple embodiments separately or in any suitable sub-combination. Moreover, although features may be described above as acting in certain combinations and even initially claimed as such, one or more features from a claimed combination can in some cases be excised from the combination, and the claimed combination may be directed to a sub-combination or variation of a sub-combination.

**Claims**

1. An ophthalmic imaging instrument (102, 120) comprising:

   a light source (200) emitting a beam of light (201);
   a polygon scanning mirror (202) comprising a plurality of reflecting facets (2021, 2022, ...);
   a driver (203) arranged to rotate during operation said polygon scanning mirror (202), such that each facet reflects the beam of light (201) at a varying angle ($\alpha$);
   an optical system (220) arranged to guide the beam (201) reflected from the polygon scanning mirror (202) towards a fundus (F) of an eye (E) of a subject through a cornea (C) of the eye (E) and to guide returning light ($L_R$) back towards the polygon scanning mirror (202);
   an aberration correction element (230) being located between the polygon scanning mirror (202) and the optical system (220), the aberration correction element (230) comprising an elongate portion (231) of varying thickness supported by a carrier (232); and
   a mask (233-1, 233-2,...) arranged on the carrier and to block light in a periphery of the elongate portion (231) of the aberration correction element (230).

2. The ophthalmic imaging instrument (102, 120) according to claim 1, wherein the periphery of the elongate portion (231) comprises areas adjacent to the longitudinal sides of said elongate portion (231) and/or areas covering side walls along the longitudinal sides of said elongate portion (231), preferably sidewalls of an elongate portion (231) that protrudes from and/or indents into the carrier (232).

3. The ophthalmic imaging instrument (102, 120) according to claim 1 or 2, wherein the elongate portion (231) of varying thickness is formed as one piece with said carrier (232).

4. The ophthalmic imaging instrument (102, 120) according to any one of claims 1 to 3, wherein the elongate portion (231) of varying thickness comprises a transmissive phase mask.

5. The ophthalmic imaging instrument (102, 120) according to any one of claims 1 to 4, wherein the mask only extends along a longer direction of the elongate portion.

6. The ophthalmic imaging instrument (102, 120) according to claim 5, wherein the mask extends along a length that is shorter than the elongate portion in the longer direction.

7. The ophthalmic imaging instrument (102, 120) according to any one of claims 1 to 6, wherein the mask comprises a coating on parts of the carrier (232), preferably on edge parts and/or side walls in relation to the elongate portion.

8. The ophthalmic imaging instrument (102, 120) according to any one of claims 1 to 7, wherein the mask comprises a coating on a sidewall of the elongate portion (231) of varying thickness.

9. The ophthalmic imaging instrument (102, 120) according to any one of claims 1 to 8, wherein the mask comprises one or more blocking elements.

10. The ophthalmic imaging instrument (102, 120) according to claim 9, wherein the blocking elements comprise sections that cover a sidewall (2313) of the elongate portion (231), an edge (2313) toward a main surface of the carrier (232), and an edge (2312) toward a bottom surface of the elongate portion (231).

11. The ophthalmic imaging instrument (102, 120) according to claim 10, wherein the blocking elements assume a form in which their respective cross-sections comprise two L-shape portions.

12. The ophthalmic imaging instrument (102, 120) according to any one of claims 9 to 11, wherein the blocking elements are located in a central region in the longitudinal direction of the elongate portion.

13. The ophthalmic imaging instrument (102, 120) according to any one of claims 1 to 12, wherein the polygon scanning mirror (202) is enclosed by a housing (208) with an opening (2080), wherein the opening (2080) is covered by the aberration correction element (230)..

14. The ophthalmic imaging instrument (102, 120) according to any one of claims 1 to 13, further comprising a detector (205) arranged to detect the returning light ($L_R$) reflected from the polygon scanning mirror (202) to generate a detection signal ($S_d$), and an access (207) to computing resources (106) arranged to generate an image (210) of the fundus (F) of the eye (E) using the detection signal ($S_d$), wherein the image (210) of the fundus (F) of the eye (E) is generated using the detection signal ($S_d$) generated from the returning light ($L_R$) reflected by all facets (2021, 2022, ...) during rotation of the polygon scanning mirror (202).

15. The ophthalmic imaging instrument (102, 120) according to any one of claims 1 to 14, wherein the ophthalmic imaging instrument (102, 120) is a scanning laser ophthalmoscope.

Fig. 1

Fig. 2

## Fig. 3A

## Fig. 3B

## Fig. 4A

## Fig. 4B

Fig. 5

Fig. 6

## EUROPEAN SEARCH REPORT

**Application Number**

EP 24 21 5102

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | EP 2 805 670 A1 (OPTOS PLC [GB]) 26 November 2014 (2014-11-26) * paragraphs [0001] - [0027]; figures 1-3 * ----- | 1-15 | INV. A61B3/10 A61B3/12 |
| Y | US 2020/400422 A1 (RALSTON TYLER S [US] ET AL) 24 December 2020 (2020-12-24) * paragraph [0175]; figure 17 * ----- | 1-15 | |
| Y | CN 112 641 423 A (BEIJING INSTITUTE TECH) 13 April 2021 (2021-04-13) * paragraph [0034]; figure 4 * ----- | 1-15 | |
| Y | US 2004/189941 A1 (BUCOURT SAMUEL HENRI [FR] ET AL) 30 September 2004 (2004-09-30) * paragraph [0028]; figure 1A * ----- | 1-15 | |
| | | | **TECHNICAL FIELDS SEARCHED (IPC)** A61B |

The present search report has been drawn up for all claims

| Place of search | Date of completion of the search | Examiner |
|---|---|---|
| Munich | 18 April 2025 | Mäki-Mantila, M |

EPO FORM 1503 03.82 (P04C01)

## ANNEX TO THE EUROPEAN SEARCH REPORT
## ON EUROPEAN PATENT APPLICATION NO.

EP 24 21 5102

This annex lists the patent family members relating to the patent documents cited in the above-mentioned European search report.
The members are as contained in the European Patent Office EDP file on
The European Patent Office is in no way liable for these particulars which are merely given for the purpose of information.

18-04-2025

| Patent document cited in search report | | Publication date | Patent family member(s) | | Publication date |
|---|---|---|---|---|---|
| EP 2805670 | A1 | 26-11-2014 | CN | 104127167 A | 05-11-2014 |
| | | | DK | 2805670 T3 | 09-01-2023 |
| | | | EP | 2805670 A1 | 26-11-2014 |
| | | | ES | 2935575 T3 | 08-03-2023 |
| | | | FI | 2805670 T3 | 31-01-2023 |
| | | | GB | 2516343 A | 21-01-2015 |
| | | | HK | 1201431 A1 | 04-09-2015 |
| | | | JP | 6681135 B2 | 15-04-2020 |
| | | | JP | 2014217756 A | 20-11-2014 |
| | | | US | 2014327882 A1 | 06-11-2014 |
| US 2020400422 | A1 | 24-12-2020 | NONE | | |
| CN 112641423 | A | 13-04-2021 | NONE | | |
| US 2004189941 | A1 | 30-09-2004 | AT | E534326 T1 | 15-12-2011 |
| | | | CN | 1568157 A | 19-01-2005 |
| | | | EP | 1427329 A2 | 16-06-2004 |
| | | | FR | 2828396 A1 | 14-02-2003 |
| | | | IL | 160347 A | 26-11-2008 |
| | | | JP | 4167979 B2 | 22-10-2008 |
| | | | JP | 2004538076 A | 24-12-2004 |
| | | | US | 2004189941 A1 | 30-09-2004 |
| | | | WO | 03015622 A2 | 27-02-2003 |

EPO FORM P0459

For more details about this annex : see Official Journal of the European Patent Office, No. 12/82